# EUROPEAN PATENT APPLICATION

(11) **EP 3 101 419 A1**
(43) Date of publication of application: **07.12.2016**
(21) Application number: 15170451.7
(22) Date of filing: 03.06.2015
(51) Int. Cl.: G01N 33/00

(54) **AN APPARATUS FOR DETECTING CARBON MONOXIDE**

(71) Applicant: Nokia Technologies Oy, 02610 Espoo (FI)
(72) Inventor: VOUTILAINEN, Martti, 02660 Espoo (FI)
(74) Representative: Nokia Corporation

(57) **Abstract**

An carbon monoxide detecting apparatus comprising: an oxidation cell configured to oxidize carbon monoxide to produce carbon dioxide; and a sensor configured to detect carbon dioxide produced by the oxidation cell.

## Description

### TECHNOLOGICAL FIELD

Embodiments of the present invention relate to an apparatus and/or method for detecting carbon monoxide.

### BACKGROUND

Existing carbon monoxide detectors have certain drawbacks.

A chemical based detector may comprise a compound that changes color in the presence of carbon monoxide. A problem with this type of detector is that it is passive and requires regular inspection.

Active carbon monoxide detectors typically convert the presence of carbon monoxide to an electrical signal which is then used to trigger an alert. These active detectors may, for example, comprise an electrochemical cell that produces an electric current by oxidizing carbon monoxide to carbon dioxide. However, such electrochemical cells require calibration and may become inaccurate if stored below zero degrees Celsius.

### BRIEF SUMMARY

According to various, but not necessarily all, embodiments of the invention there is provided an apparatus comprising: an oxidation cell configured to oxidize carbon monoxide to produce carbon dioxide; and a sensor configured to detect carbon dioxide produced by the oxidation cell.

According to various, but not necessarily all, embodiments of the invention there is provided method of carbon monoxide detection comprising, at an apparatus: oxidizing carbon monoxide to produce carbon dioxide; and detecting the produced carbon dioxide.

According to various, but not necessarily all, embodiments of the invention there is provided examples as claimed in the appended claims.

### BRIEF DESCRIPTION

For a better understanding of various examples that are useful for understanding the detailed description, reference will now be made by way of example only to the accompanying drawings in which:
Figure 1 illustrates an example of an apparatus for detecting carbon monoxide comprising an oxidation cell for oxidizing carbon monoxide to carbon dioxide and a sensor for detecting carbon dioxide;
Figure 2 illustrates another example of the apparatus;
Figure 3 illustrates an example of an oxidation cell;
Figure 4 illustrates an example of a sensor for sensing carbon dioxide;
Figure 5 illustrates an apparatus in which the oxidation cell and the sensor are integrated; and
Figure 6 illustrates an apparatus comprising a reference device.

### DETAILED DESCRIPTION

The figures illustrate an apparatus 10, for carbon monoxide 22 detection, comprising: an oxidation cell 20 configured to oxidize carbon monoxide 22 to produce carbon dioxide 24; and a sensor 30 configured to detect the carbon dioxide 24 produced by the oxidation cell 20.

The apparatus 10 separates the oxidation of the carbon monoxide 22 to carbon dioxide 24 from the detection step. Instead of attempting to directly detect carbon monoxide 22, the apparatus 10 instead converts the carbon monoxide 22 to carbon dioxide 24 and then detects the carbon dioxide 24. This enables a robust and low cost detector for carbon monoxide 22.

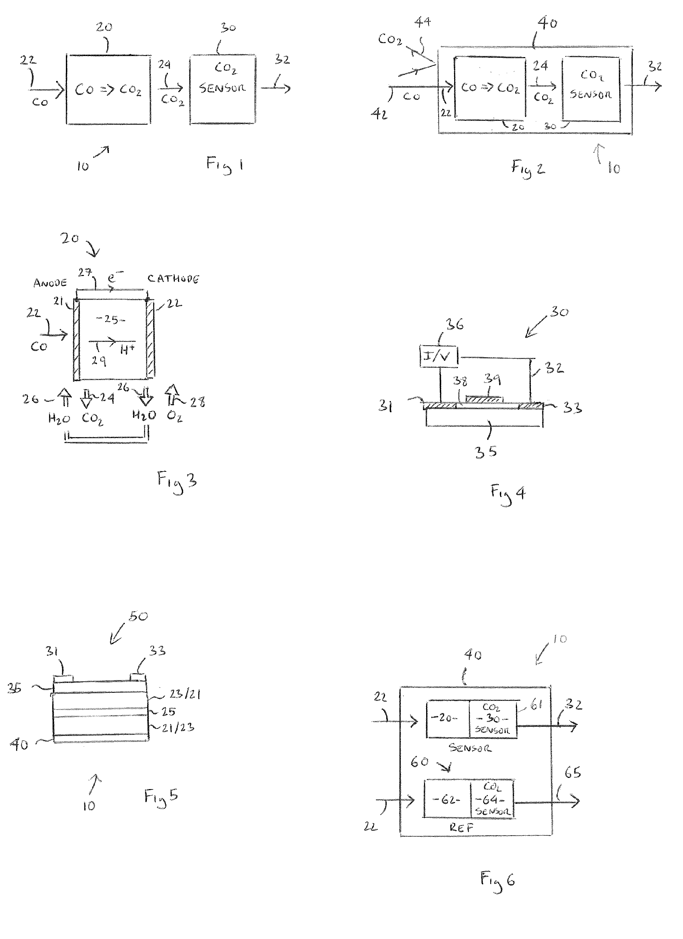

Figure 1 illustrates an example of an apparatus 10 that is configured to detect carbon monoxide 22. The apparatus 10 comprises an oxidation cell 20 and a sensor 30. The oxidation cell 20 is configured to oxidize the carbon monoxide 22 to produce carbon dioxide 24. The sensor 30 is configured to detect the carbon dioxide 24 produced by the oxidation cell 20 and provide a sensor signal 32.

In some, but not necessarily all examples it may be desirable to block, fully or partially, carbon dioxide that has not been produced by the oxidation cell 20 reaching the sensor 30 as the sensor 30 then detects proportionally more carbon dioxide 24 that has been produced as a consequence of oxidation of carbon monoxide 22.

In some, but not necessarily all examples, it may be desirable to block, fully or partially, carbon monoxide 22 from reaching the sensor 30 as it may interfere with the detection of the carbon dioxide 24 produced by the oxidation cell 20.

Figure 2 illustrates an example of the apparatus 10 that has been configured to control whether and how much external carbon dioxide 44 reaches the sensor 30 and/or to control how much external carbon monoxide 42 reaches the oxidation cell 20 and sensor 30.

In this example, the external carbon dioxide 44 may be atmospheric carbon dioxide and the apparatus 10 may be suitable for detecting atmospheric carbon monoxide 42, for example, in a home environment.

The filter 40 may be any suitable means used to control whether and how much external carbon dioxide 44 reaches the sensor 30 and/or to control how much external carbon monoxide 42 reaches the oxidation cell 20. The filter 40 may, for example, be chosen to differentially transfer external carbon monoxide 42 and external carbon dioxide 44. The filter 40 may, for example, be chosen to protect the sensor 30 from carbon monoxide by, for example, preventing the oxidation cell 20 from receiving more carbon monoxide 22 than it can oxidize. The filter 40 may, for example, be chosen to control the gain or relative change in the sensor signal 32 as a consequence of oxidation of transferred external carbon monoxide 22 to carbon dioxide 24.

For example, the filter 40 may be any suitable means used to block, fully or partially, external carbon dioxide 44 from reaching the sensor 30 whilst not substantially inhibiting external carbon monoxide 42 from reaching the oxidation cell 20. This will produce a large gain or relative change in the sensor signal 32 as a consequence of oxidation of carbon monoxide 42 to carbon dioxide 44. For example, the filter 40 may in some, but not necessarily all embodiments be a scrubber such as, for example, activated charcoal.

For example, the filter 40 may in some, but not necessarily all embodiments, be a gas permeable membrane that controls the transfer of external carbon dioxide 44 and external carbon monoxide 42. The filter 40 may in some, but not necessarily all embodiments, be a gas permeable membrane that selectively enables the transfer of external carbon dioxide 44 in preference to external carbon monoxide 42. Suitable gas permeable membranes include, for example, polyimides and other asymmetric hollow fiber membranes. An example of a soluble thermoplastic polyimide is Matrimid™ 5218. An asymmetric hollow fiber membrane may be based on polymers including cellulose acetate and polyethersulfone.

Figure 3 illustrates an example of an oxidation cell 20. In this example, the oxidation cell 20 is an electrochemical cell, however, other types of oxidation cells 20 may be used.

The electrochemical cell 20 comprises an anode 21, a cathode 23, an electrolyte 25 and an electrical current path 27 between the anode 21 and the cathode 23. Carbon monoxide 22 is oxidized at the anode 21 to produce carbon dioxide 24. The half cell reaction at the anode 21 is an oxidation reaction that consumes carbon monoxide 22 and water 26 and produces carbon dioxide 24, protons and electrons. A proton path 29 is provided from the anode 21 to the cathode 23 via the electrolyte 25 and a separate electron path is provided via the electrical path 27 from the anode 21 to the cathode 23.

The half cell reaction at the cathode 23 is a reduction reaction that consumes oxygen 28, the protons provided along the proton path 29 and the electrons provided along the electrical path 27 to produce water 26 which is re-circulated to the anode 21.

The electrical path 27 for the electrons is separate from the proton path 29 for the protons. The electrolyte 25 provides a proton path 29, but block, fully or partially, electron transfer. The electrolyte 25 is therefore preferably not electrically conductive.

The electrochemical cell operates as a redox cell. Carbon monoxide is oxidized at the anode to produce carbon dioxide 25.

The electrolyte 25 is preferably acidic so that the protons produced at the anode 21 are transferred from the anode 21 to the cathode 23.

In some, but not necessarily all examples, the electrolyte 25 may be a polymer electrolyte. Polymer electrolytes are typically solid electrolytes that provide a proton exchange membrane between the anode 21 and cathode 23. They provide for the transfer of protons from the anode 21 to the cathode 23, but are not electrically conductive. An example of a suitable polymer electrolyte is perfluorinated sulfonic acid polymer particles.

The anode 21 may comprise catalytic material such as platinum compounds. Suitable catalysts may, for example, include PtRu, PtMo, PtZn, PtSn, Pt/γ-Al₂O₃ nanoparticles. It is preferable for the catalysts to be carbon monoxide tolerant. Catalytic performance may be improved if metal oxides are included in the catalytic system. Suitable oxides include IrO₂, V₂O₅, WO₃, CeO₂, MoO₃ and RuO₂.

It may be desirable to block, fully or partially, carbon monoxide 22 from reaching the sensor 30. This may, for example, be achieved by using a carbon monoxide absorber or by using a sufficiently efficient electrochemical cell 20.

Figure 4 illustrates an example of a sensor 30 configured to detect carbon dioxide 24. The sensor 30 is a graphene-based sensor. Graphene in the presence of carbon dioxide changes its electrical properties. This change in electrical properties may be measured. The graphene-based sensor 30 may comprise as sensory material graphene or, for example, a graphene incorporated composite. One example of a graphene incorporated composite is a compound comprising graphene and yttrium oxide nanoparticles. The yttrium oxide forms quantum dots within the graphene substrate and may have a percentage by weight of approximately 20%.

In the figure, the graphene-based sensor 30 is configured as a transconductance sensor. The sensor 30 comprises a first electrode 31 separated from a second electrode 33 by a channel 35. The channel 35 is exposed to the carbon dioxide 24. Detection circuitry 36 is connected to the first electrode 31 and the second electrode 33. The detection circuitry 36 measures a change in the transconductance of the channel 35 caused by the presence of carbon dioxide 34. The change in transconductance causes a sensor signal 32 which may, for example, be an electric current that varies with the transconductance of the channel 35.

The transconductance sensor may be configured as a field effect transistor. In this implementation the channel 35 is associated with a gate electrode 38 separated from the channel 35 by a dielectric layer 39. The sensitivity of the sensor 30 may then be controlled by applying voltages to the gate electrode 38.

The graphene-based sensor 30 is not a water-based system and does not comprise components that may be damaged by sub-zero Celsius temperatures. The sensor 30 may therefore be stored at sub-zero temperatures. The oxidation cell 20 may comprise water and may comprise a proton exchange membrane that may be damaged by sub-zero temperatures. Such damage may make any calibration of a redox reaction of the electrochemical cell 20 against the transfer of electrons in the electrical path 27 unreliable, but it does not prevent the electrochemical cell 20 effectively converting all or most of the carbon monoxide 22 to carbon dioxide 24. The calibrated detection of the carbon dioxide 24 may then be performed by the sensor 30 that is not degraded or made unreliable by sub-zero temperatures.

Figure 5 illustrates another example of the apparatus 10 as previously described. It may, for example, comprise the oxidation cell 20 as illustrated in and described with reference to Figure 3 and/or it may comprise a sensor 30 as illustrated in and described with reference to Figure 4. In this example, the oxidation cell 20 and the sensor 30 are integrated into a single component 50. The oxidation cell 20 is layered comprising a filter layer 40, an electrochemical cell electrode 21/23, a proton exchange membrane 25, a further electrochemical cell electrode 23/21 that is directly interconnected to the sensor 30 which comprises a graphene-based channel 35 and electrodes 31/33.

The layered single component 50 may for example be formed by printing the different layers one on top of the other.

Figure 6 illustrates another example of the apparatus 10 as previously described. In this example, the apparatus 10 comprises a sensor device 61 the combination of the oxidation cell 20 and the sensor 30 and additionally comprises a reference device 60. The reference device 60 comprises a reference sensor 64 the same as the sensor 30 of the sensor device 61 and as previously described. The sensor 30 produces a sensor signal 32, whereas the reference sensor 64 produces a reference signal 65. The comparison of the sensor signal 32 to the reference signal 65 may improve the accuracy of detection of carbon monoxide 22.

As previously described, in the sensor device 61 the sensor 30 is associated with an oxidation cell 20 that converts carbon monoxide 22 to carbon dioxide 24 which is then detected by the sensor 30 to produce the sensor signal 32.

The reference device 60 is similar to the combination of the oxidation cell 20 and the sensor 30 in the sensor device 61 except that the oxidation cell 20 is replaced by a carbon monoxide absorber 62. The reference sensor 64 is the same as the sensor 30. The carbon monoxide absorber 62 does not convert carbon monoxide to carbon dioxide, but instead prevents the carbon monoxide reaching the reference sensor 64. The carbon monoxide absorber 62 may, for example, comprise reactive metals such as sodium, calcium, titanium, rare earth metals that react with carbon monoxide to form oxides or oxy-carbide compounds.

In this example, the filter 40 is used for both the sensor device 61 and the reference device 60. In other examples, the filter 40 may be used for only the sensor device 61 and not the reference device 60. In other examples, a filter 40 may not be used for the sensor device 61 nor the reference device 60.

The filter 40 may in some, but not necessarily all embodiments, be a gas permeable membrane that selectively enables the transfer of external carbon dioxide 44 in preference to external carbon monoxide 42 to the oxidation cell 20 of the sensor device and to the carbon monoxide absorber 62 of the reference device 60. Suitable gas permeable membranes include, for example, polyimides and other asymmetric hollow fiber membranes. An example of a soluble thermoplastic polyimide is Matrimid™ 5218. An asymmetric hollow fiber membrane may be based on polymers including cellulose acetate and polyethersulfone.

Where a structural feature has been described, it may be replaced by means for performing one or more of the functions of the structural feature whether that function or those functions are explicitly or implicitly described.

The term 'comprise' is used in this document with an inclusive not an exclusive meaning. That is any reference to X comprising Y indicates that X may comprise only one Y or may comprise more than one Y. If it is intended to use 'comprise' with an exclusive meaning then it will be made clear in the context by referring to "comprising only one.." or by using "consisting".

In this brief description, reference has been made to various examples. The description of features or functions in relation to an example indicates that those features or functions are present in that example. The use of the term 'example' or 'for example' or 'may' in the text denotes, whether explicitly stated or not, that such features or functions are present in at least the described example, whether described as an example or not, and that they can be, but are not necessarily, present in some of or all other examples. Thus 'example', 'for example' or 'may' refers to a particular instance in a class of examples. A property of the instance can be a property of only that instance or a property of the class or a property of a subclass of the class that includes some but not all of the instances in the class. It is therefore implicitly disclosed that a features described with reference to one example but not with reference to another example, can where possible be used in that other example but does not necessarily have to be used in that other example.

Although embodiments of the present invention have been described in the preceding paragraphs with reference to various examples, it should be appreciated that modifications to the examples given can be made without departing from the scope of the invention as claimed.

Features described in the preceding description may be used in combinations other than the combinations explicitly described.

Although functions have been described with reference to certain features, those functions may be performable by other features whether described or not.

Although features have been described with reference to certain embodiments, those features may also be present in other embodiments whether described or not.

Whilst endeavoring in the foregoing specification to draw attention to those features of the invention believed to be of particular importance it should be understood that the Applicant claims protection in respect of any patentable feature or combination of features hereinbefore referred to and/or shown in the drawings whether or not particular emphasis has been placed thereon.

## Claims

1. An apparatus comprising:
an oxidation cell configured to oxidize carbon monoxide to produce carbon dioxide; and
a sensor configured to detect carbon dioxide produced by the oxidation cell.

2. An apparatus as claimed in any preceding claim, wherein the sensor configured to detect carbon dioxide is a graphene-based sensor.

3. An apparatus as claimed in claim 1 or 2, wherein the sensor configured to detect carbon dioxide comprises graphene incorporated composite.

4. An apparatus as claimed in claim 2 or 3, wherein the sensor comprises first and second electrodes separated by a graphene-based channel, wherein a transconductance of the graphene-based channel is dependent upon carbon dioxide produced by the oxidation cell.

5. An apparatus as claimed in any preceding claim, further comprising a filter configured to differentially transfer atmospheric carbon monoxide and atmospheric carbon dioxide.

6. An apparatus as claimed in claim 5, wherein the filter comprises a gas permeable membrane.

7. An apparatus as claimed in any preceding claim, wherein the apparatus is configured to inhibit atmospheric carbon monoxide from reaching the sensor.

8. An apparatus as claimed in any preceding claim, wherein the oxidation cell is an electrochemical cell comprising an anode configured to oxidize carbon monoxide to produce carbon dioxide, a cathode, an electrical path between the anode and the cathode, and an electrolyte providing a proton path between the anode and the cathode separate to the electrical path.

9. An apparatus as claimed in claim 8, wherein the electrolyte is acidic.

10. An apparatus as claimed in claim 8 or claim 9, wherein the electrolyte is a polymer electrolyte.

11. An apparatus as claimed in any of claims 8 to 10, wherein the anode comprises platinum as a catalyst.

12. An apparatus as claimed in claim 11, wherein the anode further comprises metal oxide.

13. An apparatus as claimed in any preceding claim, wherein the oxidation cell and the sensor are integrated as a single component.

14. An apparatus as claimed in any preceding claim, further comprising a reference sensor configured to detect carbon dioxide and a filter configured to remove carbon monoxide from gas reaching the reference sensor, wherein the sensor configured to detect carbon dioxide produced by the oxidation cell and the reference sensor have the same configuration and wherein the reference sensor provides a reference signal and the sensor configured to detect carbon dioxide produced by the oxidation cell provides a sensor signal.

15. A method of carbon monoxide detection comprising, at an apparatus:
oxidizing carbon monoxide to produce carbon dioxide; and
detecting the produced carbon dioxide.
